# EUROPEAN PATENT APPLICATION

(11) **EP 4 428 241 A1**
(43) Date of publication of application: **11.09.2024**
(21) Application number: 22890024.7
(22) Date of filing: 04.11.2022
(51) Int. Cl.: C12P 7/625, C12N 1/20

(54) **CULTURE METHOD FOR PROMOTING PROLIFERATION AND CARBON FIXATION OF PURPLE PHOTOSYNTHETIC BACTERIA**

(30) Priority: 04.11.2021 JP 2021180569
(71) Applicant: Symbiobe Inc., Kyoto-shi, Kyoto 615-8245 (JP)
(72) Inventor: NUMATA, Keiji, Kyoto-shi, Kyoto 615-8530 (JP); FOONG, Choon Pin, Centros 138668 (SG); SRISAWAT, Pisanee, Kyoto-shi, Kyoto 615-8245 (JP); HOSHINO, Yu, Fukuoka-shi, Fukuoka 819-0395 (JP)
(74) Representative: D Young & Co LLP
(86) International application number: PCT/JP2022/041203
(87) International publication number: WO 2023/080204

(57) **Abstract**

The present invention relates to a method for producing a polyhydroxyalkanoic acid using a purple photosynthetic bacterium, including culturing the purple photosynthetic bacterium with gel particles.

## Description

### Technical Field

The present invention relates to a culture method for promoting the proliferation of purple photosynthetic bacteria and carbon fixation. Specifically, the present invention relates to a method for producing a polyhydroxyalkanoic acid by culturing a purple photosynthetic bacterium.

### Background Art

Polyhydroxyalkanoic acids (PHA), a type of bioplastic, is a substance that organisms accumulate during poor nutrition conditions, and is synthesized by numerous microorganisms. PHAs are a family of biologically synthesized polymers produced in a wide range of microorganisms for energy storage, and they are expected to be a useful environmentally-friendly, recyclable polymer material having properties such as biodegradability, marine degradability, and biocompatibility.

Furthermore, various applications of PHA have been considered, ranging from as materials in the packaging field to the biomedical field. In addition, PHA is expected to help realize sustainable development of an environmentally friendly society, replacing petrochemical-based plastics currently in mass production.

However, despite its potential to contribute to the establishment of a sustainable society, the use of PHA for purposes as described above faces the problem that raw materials, such as vegetable oils and sugars, required for producing of PHA in heterotrophs are costly.

Many previous studies have focused on producing PHA using algae, which are autotrophs that can utilize inexpensive and abundantly available resources such as sunlight and carbon dioxide (CO₂) through photosynthesis.

Furthermore, methods for producing PHA using purple photosynthetic bacteria are known, as described in Patent Literature 1 and others.

### Citation List

### Patent Literature

Patent Literature 1: JP 2018-133997 A

### Non-Patent Literature

Non-Patent Literature 1: Ishizaki, A., Tanaka, K. & Taga, N. Microbial production of poly-D-3-hydroxybutyrate from CO2. Appl. Microbiol. Biotechnol. 57, 6-12 (2001). Non-Patent Literature 2: Lau, N., Matsui, M. & Abdullah, A. A. Cyanobacteria: photoautotrophic microbial factories for the sustainable synthesis of industrial products. Biomed Res. Int. 2015, 1-9 (2015).
Non-Patent Literature 3: Claassens, N. J., Sousa, D. Z., Martins, V. A. P., Vos, W. M. De & Cost, J. van der. Harnessing the power of microbial autotrophy. Nat. Rev. Microbiol. 14, 692-706 (2016).
Non-Patent Literature 4: Kamravamanesh, D., Lackner, M. & Herwig, C. Bioprocess engineering aspects of sustainable polyhydroxyalkanoate production in Cyanobacteria. Bioengineering 111, 1-18 (2018).

### Summary of Invention

### Technical Problem

From the viewpoint of sustainable bioproduction, further improvement of PHA production techniques are desired.

The problem to be solved by the present invention is to develop and provide a method for enhancing PHA production using purple photosynthetic bacteria.

### Solution to Problem

As a result of intensive studies, the present inventors have found that the above problems can be solved by culturing purple photosynthetic bacteria with gel particles, and the present invention has been completed.

The present invention is as follows.

(1) A method for producing a polyhydroxyalkanoic acid using a purple photosynthetic bacterium, including culturing the purple photosynthetic bacterium with gel particles.
(2) The method according to (1), wherein the hydrodynamic diameter of the gel particle is 10 um or less.
(3) The method according to (1) or (2), wherein the gel particles are crosslinked bodies of a hydrated polymer.
(4) The method according to any one of (1) to (3), wherein the gel particles are anionic, cationic, or nonionic.
(5) The method according to any one of (1) to (4), wherein the gel particles are cationic.
(6) The method according to any one of (1) to (5), wherein the purple photosynthetic bacterium is a marine purple photosynthetic bacterium.
(7) The method according to (6), wherein the purple photosynthetic bacterium is selected from the group consisting of Marichromatium bheemlicum, Thiohalocapsa marina, Thiophaeococcus mangrovi, Afifella pfennigii, Afifella marina, Rhodovulum euryhalinum, Rhodovulum imhoffii, Rhodovulum sulfidophilum, Rhodovulum tesquicola, Rhodovulum visakhapatnamense, Roseospira goensis, and Roseospira marina.
(8) The method according to any one of (1) to (7), wherein the gel particles are present in a liquid culture medium.
(9) The method according to any one of (1) to (8), wherein the purple photosynthetic bacterium is cultured in a culture medium not containing an organic carbon source.
(10) The method according to any one of (1) to (9), wherein a culture medium contains a HCO₃ salt as an inorganic carbon source.

### Advantageous Effects of Invention

According to the method for producing PHA of the present invention, it is possible to enhance PHA producing by using purple photosynthetic bacteria.

### Brief Description of Drawings

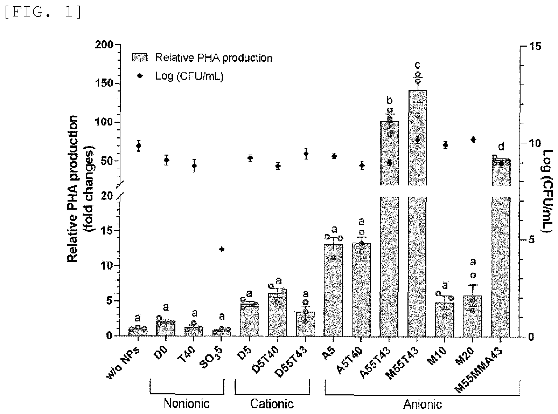

[Figure 1] Fig. 1 shows test results on the effect of gel particles on the proliferation and PHA accumulation of R. sulfidophilum. As a control experiment, culture without addition of gel particles was performed (w/o NP). Viable cells (shown in black dot plots) and relative PHA production (shown in gray bars) were observed over 72 hours of culture. The relative PHA production amount was calculated with respect to the PHA accumulation amount in the control experiment. Cultures were performed at n = 3 and error bars indicate the standard error of the mean (S.E.) values. Different letters indicate significantly different p values < 0.05 for PHA concentration.

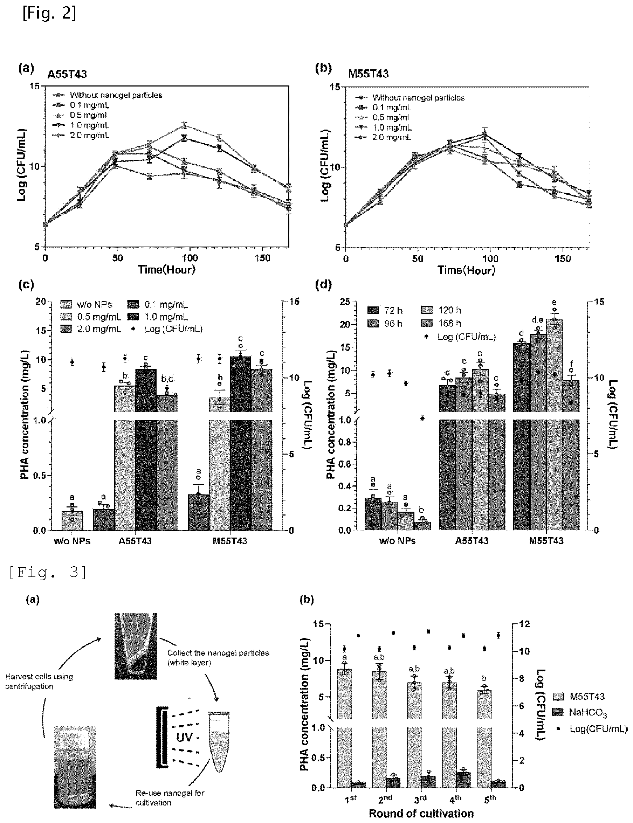

[Figure 2] Fig. 2 shows the CFU profile (2a, 2b) and PHA production (2c, 2d) of R. sulfidophilum cultured in the presence of A55T43 gel particles and M55T43 gel particles in photoautotrophic culture. The initial culture (at 0 hours) was standardized to an optical density (OD₆₆₀) of 0.1 and taken as 6.5 log (CFU/mL). Fig. 2c shows a bar graph of PHA production in 72 hours of photoautotrophic culture under different concentrations (in the range of 0.1 to 2 mg/mL). The log (CFU/mL) represents the number of viable cells at the time of collection in a dot plot. Fig. 2d shows PHA production after 72 hours (leftmost bar), 96 hours (second bar from left), 120 hours (second bar from right), 168 hours (rightmost bar) of photoautotrophic culture with addition of 1 mg/mL of gel particles. The dot plot shows the number of viable cells at the time of collection in log (CFU/ml). Cultures were performed at n = 3 and error bars indicate the standard error of the mean (S.E.) values. Different letters indicate significantly different p values < 0.05 (n = 3) for PHA concentration.
[Figure 3] Fig. 3 shows the reusability of M55T43 gel particles for R. sulfidophilum culture. Fig. 3a is a cycle diagram showing that the M55T43 gel particles were separated from the culture broth using centrifugation and the gel particle layers were collected and subjected to UV sterilization prior to the next cycle of R. sulfidophilum culture. Fig. 3b shows PHA production after 72 hours of photoautotrophic culture using M55T43 gel particles repeated 5 times (left bar). Control experiments were performed at each round in R. sulfidophilum cultures under NaHCO₃ (right bar). The dot plot shows the number of viable cells at the time of collection in log (CFU/mL). Cultures were performed at n = 3 and error bars indicate the standard error of the mean (S.E.) values.

### Description of Embodiments

The present invention is a method for producing a polyhydroxyalkanoic acid using purple photosynthetic bacteria. The method for producing a polyhydroxyalkanoic acid of the present invention is preferably carried out by performing a culture method for promoting proliferation of purple photosynthetic bacteria and carbon fixation.

The production method includes a step of culturing purple photosynthetic bacteria with gel particles.

In the present invention, the biosynthesis of polyhydroxyalkanoic acid can be enhanced by culturing purple photosynthetic bacteria with gel particles.

The purple photosynthetic bacteria used in the present invention mean purple sulfur bacteria and purple non-sulfur bacteria.

Purple sulfur bacteria are bacteria which grow photoautotrophically in the presence of hydrogen, sulfide, and carbon dioxide and perform photosynthesis utilizing near-infrared light, and purple non-sulfur bacteria are photosynthetic bacteria which grow photoheterotrophically in the presence of organic substances and the like.

The purple photosynthetic bacteria can be roughly classified into freshwater purple photosynthetic bacteria and marine purple photosynthetic bacteria depending on the habitat, but marine purple photosynthetic bacteria are preferably used. According to the method of the present invention, purple non-sulfur bacteria can be used, and marine purple non-sulfur bacteria may also be used.

Examples of the purple photosynthetic bacteria include, for example, purple sulfur bacteria such as bacteria belonging to the genus Allochromatium (also described as Allochromatium sp., the same applies hereinafter), the genus Ectothiorhodospira, the genus Halochromatium, the genus Halorhodospira, the genus Marichromatium, the genus Thiocapsa, the genus Thiohalocapsa, and the genus Thiophaeococcus, and the like, and purple non-sulfur bacteria such as bacteria belonging to the genus Rhodobaca, the genus Rhodobacter, the genus Rhodobium, the genus Afifella (Rhodobium), the genus Rhodothalassium, the genus Rhodovulum, the genus Roseospira, and the like.

Among them, marine purple sulfur bacteria such as Marichromatium bheemlicum, Thiohalocapsa marina and Thiophaeococcus mangrovi, and marine purple non-sulfur bacteria such as Afifella pfennigii (Rhodobium pfennigii), Afifella marina (Rhodobium marinum), Rhodovulum euryhalinum, Rhodovulum imhoffii, Rhodovulum sulfidophilum, Rhodovulum tesquicola, Rhodovulum visakhapatnamense, Roseospira goensis, and Roseospira marina are preferably used.

These purple photosynthetic bacteria may be obtained from respective depositories by a predetermined procedure.

In addition, as the purple photosynthetic bacteria, bacteria disclosed in PLOS ONE | DOI:10.1371/journal.pone.0160981 may be used, and specific examples thereof include the following purple photosynthetic bacteria disclosed as Table 1 in the paper. In Table 1 below, specific bacteria are described as the Resource No. of the purple photosynthetic bacteria, but the bacteria belonging to the same genus or the same species as the purple photosynthetic bacteria described in Table 1 may be used. The purple photosynthetic bacteria described as Organism in Table 1 below may be used, and specific examples thereof may be Thiohalocapsa marina, Thiophaeococcus mangrovi, Marichromatium bheemlicum, Afifella marina, Rhodovulum euryhalinum, Rhodovulum imhoffii, Rhodovulum sulfidophilum, Rhodovulum tesquicola, Rhodovulum visakhapatnamense, Roseospira marina, and Roseospira goensis, and when the classification is changed and the name of the bacteria is changed, the purple photosynthetic bacteria as the name of the new bacteria may be used. In addition, the bacteria belonging to the genus Thiohalocapsa, the genus Thiophaeococcus, the genus Marichromatium, the genus Afifella, the genus Rhodovulum, the genus Roseospira, and the genus Roseospira, which are the generic names thereof may be used.

[Table 1]

| **Sulfur type** | **Resource No.** | **Organism** | **Original marine area** |
|---|---|---|---|
| Sulfur | DSM5653 | *Thiohalocapsa marina* | Mediterranean Sea |
| Sulfur | JCM14889 | *Thiophaeococcus mangrovi* | Orissa, India |
| Sulfur | JCM13911 | *Marichromatium bheemlicum* | Bhimunipatnam, India |
| Non-sulfur | DSM2698 | *Afifella marina* | Kagoshima, Japan |
| Non-sulfur | DSM4868 | *Rhodovulum euryhalinum* | Russia |
| Non-sulfur | JCM13589 | *Rhodovulum imhoffii* | Bhimunipatnam, India |
| Non-sulfur | ATCC35886 | *Rhodovulum sulfidophilum* | Groningen, Netherlands |
| Non-sulfur | ATCC BAA1573 | *Rhodovulum tesquicola* | Soda Lake, Russia |
| Non-sulfur | JCM13531 | *Rhodovulum visakhapatnamense* | Visakhapatnam, India |
| Non-sulfur | ATCC BAA447 | *Roseospira marina* | Arcachon Bay, France |
| Non-sulfur | JCM14191 | *Roseospira goensis* | Goa, India |
| Non-sulfur | ATCC BAA1365 | *Roseospira visakhapatnamensis* | Kakinada, India |

| | | | |
|---|---|---|---|
| doi:10.13711journal.pone.0160981.t001 | | | |

R. sulfidophilum, a marine photosynthetic bacterium capable of proliferating under photoheterotrophic or photoautotrophic conditions, may be used. In the present invention, R. sulfidophilum or similar marine photosynthetic bacteria may be cultured under photoautotrophic conditions.

The polyhydroxyalkanoic acid (PHA) produced in the present invention can be represented by the following general formula I. 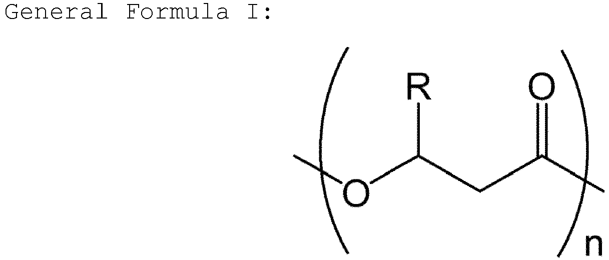 [wherein R may be the same or different, and is a linear alkyl group having 1 to 14 carbon atoms or an alkyl group having a branched chain, and n is an integer of 2 or more.]

PHA is a substance that can be understood as a polyester of a hydroxyalkanoic acid represented by the following general formula II. 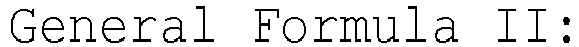 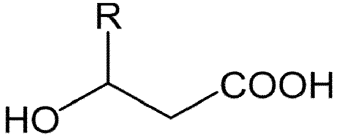 [wherein R is a linear alkyl group having 1 to 14 carbon atoms or an alkyl group having a branched chain.]

In general formula I, it is also preferred that the polyester be derived from the polyester of hydroxyalkanoic acid represented by the following general formula III. 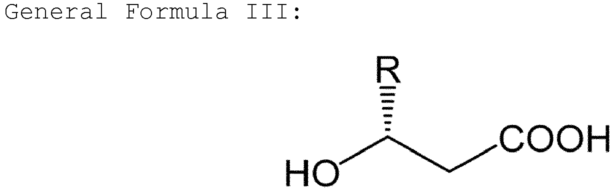 [wherein R is a linear alkyl group having 1 to 14 carbon atoms or an alkyl group having a branched chain.]

Specific examples of the PHA are not particularly limited, but for example, in the general formula I, the number of carbon atoms is preferably 1 to 3.

The PHA may be polyhydroxybutyrate, polyhydroxyvalerate (PHV: P (3HV)) (Poly-3-hydroxyvalerate), or polyhydroxyhexanoate.

The hydroxyalkanoic acid constituting the PHA may be a single hydroxyalkanoic acid or a copolymer of two or more kinds of hydroxyalkanoic acids.

n is not particularly limited, but is, for example, preferably an integer of 100 or more and preferably an integer of 100,000 or less.

The gel particles used in the present invention are polymer compound particles, and for example, materials described in WO 2013/027668 and WO 2016/024633 can be used. The gel particles are preferably adjusted in pKa.

The gel particles may be anionic, cationic, or nonionic, but are preferably anionic or cationic, and more preferably cationic.

Among the cationic gel particles, A55T43 and M55T43 gel particles can be suitably used.

In the present invention, it is considered that the gel particles are preferably capable of absorbing CO₂ or capturing salt ions.

Although not particularly limited, and exemplarily used for description, in the A55T43 and M55T43 gel particles, N-tert-butylacrylamide (TBAm) which is a monomer thereof forms a high-density core structure, and acrylic acid (AAc) and methacrylic acid (MAc) which are comonomers thereof are mostly located on the shell of the particles to form a hydrophilic surface. It is believed that the weakly acidic groups on the surface, along with the ability to capture salt ions, create a stress microenvironment within the R. sulfidophilum cultures. It is believed that the stress microenvironment created by the addition of gel particles enhances PHA accumulation in photoautotrophic culture.

The contents described in the references cited herein, particularly in the references cited for gel particles, are incorporated by reference as a description herein.

The gel particles can be polymers of monomers containing acidic groups (for example, sulfuric acid groups, carboxylic acid groups, phosphoric acid groups, phenolic hydroxyl groups, and the like) and/or basic groups (for example, amino groups, imidazole groups, pyridyl groups, and the like). The monomers described in WO 2013/027668 and WO 2016/024633 may be used.

Examples of the monomer include N-isopropylacrylamide, N-[3-(dimethylamino) propyl] methacrylamide, N-tert-butylacrylamide, N, N'-methylenebis (acrylamide), (meth) acrylic acid esters such as acrylic acid, methacrylic acid, and methyl methacrylate, and 2-acrylamido-2-methyl-1-propanesulfonic acid, and the monomer may be a copolymer thereof.

The pKa of the copolymer can be appropriately adjusted, and for example, the pKa may be 6 to 9, 7 to 8, or 6 to 7.

The copolymer may be a copolymer of any combination of the above monomers, but may be a copolymer of 3 or 4 monomers selected from N-isopropylacrylamide, N-[3-(dimethylamino) propyl] methacrylamide, N-tert-butylacrylamide, N, N'-methylenebis (acrylamide), and acrylic acid.

The copolymer may be a copolymer of N-alkylacrylamide such as N-tert-butylacrylamide with acrylic acid and/or methacrylic acid or an ester thereof. The copolymer may be a copolymer with other monomers, or a copolymer composed of 35 to 50 mol% of N-tert-butylacrylamide and 60 to 35 mol% of acrylic acid and/or methacrylic acid or an ester thereof. When other components are contained, 0 to 5 mol% of other monomers may be contained.

The copolymer may be a copolymer with a crosslinkable monomer as a crosslinking agent. By using the crosslinkable monomer, the gel particles can be crosslinked polymer compound particles (gel particles having a crosslinked structure), and preferably may be a crosslinked body of a hydrated polymer.

The crosslinkable monomer is not particularly limited as long as it is a monomer capable of forming a crosslinked structure, but N, N'-alkylenebis (acrylamide) may be preferably used (the number of carbon atoms of alkylene may be 1 to 12, 1 to 4, 1 to 3, or 1 to 2), and among these, N, N'-methylenebis (acrylamide) can be suitably used.

The crosslinkable monomer may be contained in an amount of 0 to 5 mol% or 0.1 to 5 mol%.

A crosslinking agent that is not a crosslinking monomer may be used, and the swellability of the particles can be controlled by forming a crosslinked structure in the polymer compound in the gel particles so that the particles do not swell excessively.

The size of the gel particles is preferably 10 µm or less, more preferably several µm or less in diameter, and the size of several µm or less may be 5 µm or less, 4 µm or less, 3 µm or less, 2 µm or less, or 1 µm or less.

The size of the gel particles may be 10 nm or more or 20 nm or more in diameter.

The size of the gel particles may be on the order of nm, and may be particles called nanogel particles.

The diameters of the gel particles may be from 10 nm to 10 pm, from 10 nm to several µm (the several µm may be 5 µm, 4 µm, 3 µm, 2 µm, or 1 µm), from 10 to 800 nm, from 20 to 800 nm, from 50 to 400 nm, from 80 to 300 nm, or from 100 to 200 nm.

Regarding the diameter, as described in Examples, the "particle diameter after swelling in water" is a particle diameter after immersing dried gel particles in water for 24 hours, and refers to an average particle diameter measured by a dynamic light scattering method.

The method for producing PHA of the present invention includes a culturing step as an essential step.

In the culturing step, PHA is produced by culturing purple photosynthetic bacteria with gel particles.

The production of PHA utilizes PHA producing capacity as a bio factory possessed by purple photosynthetic bacteria.

The method for producing PHA may include a PHA extraction step and a gel particle recovery step in addition to the culturing step.

As used herein, the term "culturing step" means a step of culturing purple photosynthetic bacteria under specific conditions to increase the number of bacterial cells and accumulate PHA in the bacterial cells.

In the culturing step of the present invention, it is preferable that the proliferation of the purple photosynthetic bacteria be achieved by culturing the purple photosynthetic bacteria under specific conditions.

In the present invention, the presence of gel particles is essential in the culturing step.

The culture is preferably performed in a liquid culture medium, and in the culture in the liquid culture medium, it is preferable that the liquid culture medium be physically stirred, but it is not particularly limited.

The amount of the gel particles present in the culturing step is not particularly limited, but the gel particles are preferably contained in a concentration range of 0.01 to 100 mg/mL. For example, it may be 0.05 to 30 mg/mL, 0.05 to 20 mg/mL, or 0.05 to 10 mg/mL.

The culture may be performed under irradiation of near-infrared light utilized by the purple photosynthetic bacteria to grow photoautotrophically, or may be performed under irradiation of far-infrared light.

The far-infrared light may be light having a peak wavelength in a wavelength range of 700 nm to 860 nm.

The irradiation method of the far-infrared light is not particularly limited, and a conventional irradiation method for culturing purple photosynthetic bacteria may be utilized.

The culture time may be sufficient to accumulate the PHA in the purple photosynthetic bacteria, and the culture temperature can be appropriately set according to the optimum culture temperature of the purple photosynthetic bacteria.

The culture medium used for culture is not particularly limited as long as it is a culture medium capable of culturing the purple photosynthetic bacteria, and a conventionally known proliferation culture medium may be used.

The proliferation culture medium may contain an organic carbon source, but in the present invention, PHA can be produced even when the proliferation culture medium contains an inorganic carbon source. When an inorganic carbon source is contained, the culture medium may be free of an organic carbon source.

The culture medium used for culture is not particularly limited, but natural seawater may be used, or a seawater culture medium utilizing natural seawater may be used.

In the present invention, carbon fixation is preferably promoted by culturing in a culture medium in which an inorganic carbon source is preferably used, and in some cases, an organic carbon source is not used.

The PHA obtained by the production method of the present invention may be recovered together with bacterial cells from a culture medium, or may be recovered by destroying the bacterial cells.

The gel particles used in the culturing step of the present invention can be reused by being recovered from the culture medium.

The gel particles may be separated from the culture medium by centrifugation or the like, and the washed gel particles may be subjected to culture again.

### Examples

Hereinafter, the present invention will be described in more detail with reference to Examples, but the scope of the present invention is not limited to these Examples.

### (Synthesis of Gel Particles)

Gel particles were synthesized with the monomer composition of Table 2 using the protocols described in References 1 and 2 below. The gel particles stock solution was prepared to have a concentration of 20 to 25 mg/mL in purified water, subjected to UV sterilization, and used for culture.

Each monomer in Table 2 means the following.
NIPAm: N-isopropylacrylamide
DMAPM: N-[3-(Dimethylamino)propyl]methacrylamide
TBAm: N-tert-butylacrylamide
BIS: N,N'-methylenebis(acrylamide)
AAc: Acrylic acid
MAc: Methacrylic acid
MMA: Methyl methacrylate
AMPS: 2-Acrylamido-2-methyl-1-propanesulfonic acid.

Various physical properties of the gel particles were measured according to the methods described in References 1 and 2. The diameter of the gel particles was measured as a hydrodynamic diameter after swelling in water, specifically as a hydrodynamic diameter in water measured by a dynamic light scattering method under the temperature conditions described in Table 2.

**[Table 2] [0037]**

| Nano particle | | Monomer(mol%) | | | | | | | | Diameter | | pKa (30 °C) | Carboxylic acid in NPs (mmol/g) | Amine in NPs (mmol/g) |
|---|---|---|---|---|---|---|---|---|---|---|---|---|---|---|
| | | NIPAm | DMAPM | TBAm | BIS | AAc | MAc | MMA | AMPS | 30 °C | 75 °C | | | |
| Nonionic | D0 | 98 | - | - | 2 | - | - | - | - | 113 | 74 | | | |
| | T40 | 58 | - | 40 | 2 | - | - | - | - | 89 | 91 | | | |
| Cationic | D5 | 93 | 5 | - | 2 | - | - | - | - | 220 | 87 | 7.84 | | 0.402 |
| | D5T40 | 53 | 5 | 40 | 2 | - | - | - | - | 155 | 91 | 7 | | 0.401 |
| | D55T43 | - | 55 | 43 | 2 | - | - | - | - | 359 | 268 | 7.53 | | 3.22 |
| Anionic | A5 | 93 | - | - | 2 | 5 | - | - | - | 132 | 54 | 6.45 | 0.556 | |
| | A5T40 | 53 | - | 40 | 2 | 5 | - | - | - | 121 | 118 | 6.38 | 0.515 | |
| | A55T43 | - | - | 43 | 2 | 55 | - | - | - | 152 | 153 | 6.1 | 4.55 | |
| | M55T43 | - | - | 43 | 2 | - | 55 | - | - | 110 | 112 | 69 | 593 | |
| | M10 | 88 | - | - | 2 | - | 10 | | - | 236 | 157 | 6.27 | 0.471 | |
| | M20 | 78 | - | - | 2 | - | 20 | - | - | 231 | 102 | 6.06 | 1393 | |
| | M55MMA43 | - | - | - | 2 | - | 55 | 43 | - | 189 | 188 | 8.7 | 5.458 | |
| | SO₃5 | 93 | - | - | 2 | - | - | - | 5 | 221 | 132 | | 0.383 | |

As shown in Table 2, as the gel particles, nonionic gel particles (D0 and T40), cationic gel particles (D5, D5T40, and D55T43), and anionic gel particles (A5, A5T40, A55T43, M55T43, M10, M20, M55MMA43, and SO₃5) were used.

### (Culture medium composition and culture of photosynthetic bacteria)

Rhodovulum sulfidophilum DSM1374/ATCC35886/W4 was obtained from the American Type Culture Collection (ATCC) Biological Resource Center (BRC).

R. sulfidophilum was cultured in M6 culture medium containing the following composition (with 2% NaCl added) per 1 L.

5 g of sodium malate; KH₂PO₄, 0.75 g; K₂HPO₄, 0.78 g; CaCl₂·2H₂O, 0.029 g; MgSO₄·7H₂O, 0.247 g; (NH₄)₂SO₄, 1g; FeSO₄·7H₂O, 0.011 g; 10 mL of vitamin solution; 10 µL of trace element solution

The composition of the vitamin solution per 100 mL was 0.1 g of nicotinic acid, 0.1 g of thiamine, 0.005 g of biotin, 0.05 g of para-aminobenzoic acid, 0.001 g of vitamin B₁₂, 0.05 g of vitamin B₅, 0.05 g of pyridoxine hydrochloride, 0.2 g of EDTA-3Na, 0.05 g of folic acid, 70 µg of ZnCl₂·5H₂O, 100 µg of MnCl₂·4H₂O, 60 µg of H₃BO₃, 200 ug of CoCl₂·6H₂O, 20 ug of CuCl₂·2H₂O, 20 µg of NiCl₂·6H₂O, and 40 µg of Na₂MoO₄·H₂O.

The composition of the trace element per 1 L was 11.16 g of MnSO₄·4H₂O, 2.88 g of ZnSO₄·7H₂O, 2.92 g of Co(NO₃)₂·6H₂O, 2.52 g of CuSO₄·5H₂O, 2.42 g of Na₂MoO₄·2H₂O, 10 g of H₃BO₃, and 41.20 g of EDTA·3Na.

The pH of the culture medium was adjusted to 7.0 prior to autoclave sterilization.

For inoculum culture preparation, one agar culture colony of R. sulfidophilum was cultured in 50 mL of M6 culture medium in a sterile screw-cap tube.

Cultures were maintained in static conditions at 30°C under continuous far-red LED light (730 nm, about 20Wm⁻², VBP-L24-C3, Valore) until optical cell density at 660 nm absorbance (OD₆₆₀) reached about 1 to 1.5 (intermediate logarithmic phase). The cultures were then transferred to carbon-free M6 culture medium containing 20mM NaHCO₃ (M6 culture medium without sodium malate) and maintained at the same conditions until the cells reached the intermediate logarithmic phase.

For photoautotrophic culture of R. sulfidophilum with gel particles, the seed culture was inoculated into carbon-free M6 culture medium containing 20mM NaHCO₃. Initial cultures were standardized at OD₆₆₀ = 0.1, corresponding to 6.5 Log (CFU/mL), in sterile screw-cap vials with silicone septa. The UV sterilized gel particles were added in a concentration range of 0.1 to 2 mg/mL. The cultures were then bubbled with nitrogen gas for 5 to 10 minutes to maintain the same culture conditions as described above.

### (Growth profile of R. sulfidophilum)

The time course profile of R. sulfidophilum cell proliferation was observed at 24 intervals using the plate count method. As a measurement of the number of colony forming units (CFU), the culture broth was diluted by making serial dilutions and 100 µL of L culture broth was diluted with 900 µL of carbon-free M6 culture medium. Next, 100 µL of the mixture was collected and mixed with 900 µL of carbon-free M6 culture medium. This operation was repeated to obtain the number of cells suitable for plate counting.

Next, 100 µL of the diluted culture solution was spread on a marine agar culture medium (BD Difco) plate, and incubated under far-red LED light at 30°C for 48 to 60 hours. The number of colonies on the plate was counted, and multiplied by the dilution factor to calculate the number of CFU per 1mL (log (CFU/mL)).

### (Quantification of PHA)

The cultures on days 0, 3, 4, 5, and 7 were collected by centrifugation at 8000 × g at 4°C for 10 minutes, washed twice with distilled water, maintained at - 80°C overnight, and then lyophilized for 24 to 48 hours. The method for determining the PHA content was according to the methods described in 10, 25, 31, and 32 described in References 3 to 6 below. The lyophilized cells were subjected to methanolysis with [1:1 chloroform and methanolysis solution (methanol: sulphuric acid, 85:15 v /v)] at 100°C for 180 minutes. After cooling to room temperature, phosphate buffer (pH 8) was added to the reaction mixture and neutralized with 1N NaOH. The chloroform layer was transferred to a glass tube containing anhydrous sodium sulfate to remove water.

The PHA content was measured using a gas chromatography-mass spectrometry (GCMS)(GCMS-QP2010 Ultra, Shimadzu) equipped with a 30 m × 0.25 mm × 0.25 um DB-1 capillary gas chromatography column (Agilent Technologies). For analysis, the injection temperature was set to 250°C with splitless injection and the interface temperature was set at 230°C. The column temperature program used for the separation of the ethyl ester was up to 117°C with a temperature ramp of 45°C, 1 min, 7°C/min. Helium was used as a carrier gas at a flow rate of 3.30 mL/min. The PHA content was quantified using a standard calibration curve.

### (Isotopic analysis of proteinaceous amino acids from ¹³C-tracer experiments)

Tracer experiments for ¹³C were performed with [¹³C] NaHCO₃ (purity 99 atom%, Cambridge Isotope Laboratories). For photoautotrophic proliferation, 20mM [¹³C] NaHCO₃ was added to carbon-free M6 culture medium. The seed culture in the intermediate logarithmic phase was used to inoculate carbon-free M6 culture medium containing labeled substrate to an initial OD₆₆₀ = 0.1. UV sterilized gel particles were added at a concentration of 1.0 mg/mL and the cultures were bubbled with nitrogen gas for 5 to 10 minutes. The cultures were maintained in static conditions at 30°C under continuous far-red LED light (730 nm, about 20 Wm⁻²). Cultures were performed twice. The measurement samples were collected at the initial timing (within 10 minutes after addition of [¹³C] NaHCO₃) and at the end of the exponential phase (72 hours of culture). The samples were collected at a volume corresponding to OD₆₆₀ = 3, pelleted by centrifugation at 8,000 × g for 10 min at 4°C, washed twice with distilled water, and maintained at -80°C until analysis.

Isotopic analysis of proteinaceous amino acids was performed using a chromatography-mass spectrometry (GC-MS) according to the methods described in References 7 to 9 below.

### (Statistical analysis)

Comparison of mean values between different groups was performed using one way analysis of variance (ANOVA) followed by Tukey's multiple comparison test with a statistical significance level of p < 0.05. All statistical analyses were performed with GraphPad Prism version 9.1.0 from MacOS (GraphPad Software).

### (Screening of effect of gel particles on R. sulfidophilum culture)

The pH of the culture after the addition of gel particles was adjusted to pH 7 (30°C), which is the optimal proliferation condition for R. sulfidophilum. To avoid absorbance interference due to the addition of gel particles, viable plate counts were used instead of optical cell densities using OD₆₆₀ to observe the effect on cell proliferation.

Based on the colony forming units observed at 72 hours of co-culture, none of the gel particles had a negative effect on the proliferation of R. sulfidophilum cells.

Subsequently, the effect of gel particle addition on polyhydroxyalkanoic acid (PHA) accumulation in R. sulfidophilum under photoautotrophic culture was analyzed. PHA was extracted from R. sulfidophilum cell pellets harvested after 72 hours of co-culture, and the amount of PHA was estimated using GC-MS.

The results are shown in Fig. 1. Among them, the addition of the anionic gel particles of A55T43, M55T43, and M55MMA43 increased PHA accumulation 102-fold, 142-fold, and 53-fold, respectively, as compared to the control (w/o NP) group.

### (Proliferation and PHA accumulation of R. sulfidophilum under photoautotrophic culture with gel particles added)

The effect of addition of A55T43 and M55T43 gel particles on photoautotrophic growth was evaluated. R. sulfidophilum was cultured under NaHCO₃ added as the sole carbon source with or without addition of various concentrations (0.1 to 2.0 mg/mL) of gel particles in batch culture conditions. Cell proliferation was monitored over time using viable plate counts.

The results are shown in Fig. 2.

Based on the time course profile of colony forming unit (CFU) count, the addition of A55T43 gel particles in the range of 0.1, 0.5, 1.0, and 2.0 mg/mL resulted in a slow increase in the number of CFU, which was then stable at approximately 10.5 to 11 log (CFU/mL) until 72 hours compared to the the initial 48 hours of control (w/o NP) group (Fig. 2a).

There was a gradual increase in the addition of M55T43 gel particles with a similar trend at all concentrations tested (0.1, 0.5, 1.0, and 2.0mg/mL) compared to the control (no NP) group up to 72 hours (Fig. 2b).

PHA accumulation in R. sulfidophilum grown under photoautotrophic culture containing various concentrations of A55T43 and M55T43 gel particles was analyzed after 72 hours of co-culture. A dramatic increase in PHA accumulation was observed at the addition of 0.5, 1.0, and 2.0 mg/mL concentrations of gel particles compared to the control (w/o NP) group (Fig. 2c).

PHA accumulation in R. sulfidophilum grown in photoautotrophic culture with A55T43 and M55T43 gel particles at a concentration of 1.0 mg/mL was then analyzed in 168. Sampling was performed for PHA measurement at after 72, 96, 120, and 168 hours of co-culture. There was a continuous increase until 120 hours of culture (Fig. 2d).

### (Reusability of M55T43 gel particles for R. sulfidophilum culture)

It was observed that the M55T43 gel particles could be separated after culture using centrifugation (Fig. 3a). The gel particles used were separated from the cell pellet, UV sterilized and then added to the next cycle of R. sulfidophilum culture to perform the culture (Fig. 3a). Cultures were performed using NaHCO₃ as the sole carbon source. PHA accumulation in R. sulfidophilum cells was observed at 5 culture cycles with repeated use of M55T43 gel particles.

The results are shown in Fig. 3.

It was confirmed that the desired PHA was accumulated in the cells by the method for producing PHA of the present invention.

This study demonstrated the application of gel particles for bio-producing where microorganisms are used as cell factories. PHA accumulation in R. sulfidophilum under photoautotrophic conditions was successfully enhanced up to 142-fold. In addition, the gel particles could maintain the ability to enhance PHA production with respect to at least 5 cycles of R. sulfidophilum culture. The results of this study demonstrate that PHA production by gel particles under photoautotrophic culture in R. sulfidophilum is enhanced, and the method for producing PHA according to the present invention can provide a sustainable bio-producing technique.

### References

1 Hoshino, Y. et al. Combining acid- and base-imprinted nanoparticles in a hydrogel film for temperature-responsive quick and reversible capture of salt. Appl. Polym. Mater. 2, 505-514 (2020).
2 Hoshino, Y., Imamura, K., Yue, M., Inoue, G. & Miura, Y. Reversible absorption of CO2 triggered by phase transition of amine-containing micro- and nanogel particles. J. Am. Chem. Soc. 134, 18177-18180 (2012).
3 Li, Z., Yang, J. & Loh, X. J. Polyhydroxyalkanoates: opening doors for a sustainable future. NPG Asia Mater. 8, 1-20 (2016).
4 Foong, C. P., Higuchi-takeuchi, M. & Numata, K. Optimal iron concentrations for growth- associated polyhydroxyalkanoate biosynthesis in the marine photosynthetic purple bacterium Rhodovulum sulfidophilum under photoheterotrophic condition. PLoS One 14, 1-12 (2019).
5 Higuchi-takeuchi, M., Morisaki, K. & Numata, K. A screening method for the isolation of polyhydroxyalkanoate-producing purple non-sulfur photosynthetic bacteria from natural seawater. Front. Microbiol. 7, 1-7 (2016).
6 Higuchi-takeuchi, M., Motoda, Y., Kigawa, T. & Numata, K. Class I polyhydroxyalkanoate synthase from the purple photosynthetic bacterium Rhodovulum sulfidophilum predominantly exists as a functional dimer in the absence of a substrate. ACS Omega 2, 5071-5078 (2017).
7 Osanai, T. et al. Capillary electrophoresis - mass spectrometry reveals the distribution of carbon metabolites during nitrogen starvation in Synechocystis sp. PCC 6803. Environ. Microbiol. 16, 512-524 (2014).
8 Osanai, T., Shirai, T., Iijima, H., Nakaya, Y. & Okamoto, M. Genetic manipulation of a metabolic enzyme and a transcriptional regulator increasing succinate excretion from unicellular cyanobacterium. Front. Microbiol. 6, 1-10 (2015).
9 Osanai, T. et al. Pathway-level acceleration of glycogen catabolism by a response regulator in the cyanobacterium Synechocystis species PCC 6803. Plant Physiol. 164, 1831-1841 (2014).

## Claims

1. A method for producing a polyhydroxyalkanoic acid using a purple photosynthetic bacterium comprising
culturing the purple photosynthetic bacterium with a gel particles.

2. The method according to claim 1, wherein the hydrodynamic diameter of the gel particle is 10 um or less.

3. The method according to claim 1 or 2, wherein the gel particles are crosslinked bodies of a hydrated polymer.

4. The method according to any one of claims 1 to 3, wherein the gel particles are anionic, cationic or nonionic.

5. The method according to any one of claims 1 to 3, wherein the gel particles are cationic.

6. The method according to any one of claims 1 to 5, wherein the purple photosynthetic bacterium is a marine purple photosynthetic bacterium.

7. The method according to claim 6, wherein the purple photosynthetic bacterium is selected from the group consisting of Marichromatium bheemlicum, Thiohalocapsa marina, Thiophaeococcus mangrovi, Afifella pfennigii, Afifella marina, Rhodovulum euryhalinum, Rhodovulum imhoffii, Rhodovulum sulfidophilum, Rhodovulum tesquicola, Rhodovulum visakhapatnamense, Roseospira goensis and Roseospira marina.

8. The method according to any one of claims 1 to 7, wherein the gel particles are present in a liquid culture medium.

9. The method according to any one of claims 1 to 8, wherein the purple photosynthetic bacterium is cultured in a culture medium not comprising an organic carbon source.

10. The method according to any one of claims 1 to 9, wherein a culture medium comprises a HCO₃ salt as an inorganic carbon source.
